# EUROPEAN PATENT APPLICATION

(11) **EP 4 485 004 A1**
(43) Date of publication of application: **01.01.2025**
(21) Application number: 23187196.3
(22) Date of filing: 24.07.2023
(51) Int. Cl.: G01S 7/52, G01S 15/89, A61B 8/00, A61B 8/08

(54) **SPATIAL COMPOUNDING IN ULTRASOUND IMAGING**

(30) Priority: 30.06.2023 US 202363524339 P
(71) Applicant: Koninklijke Philips N.V., 5656 AG Eindhoven (NL)
(72) Inventor: ALVA, Ashley, Eindhoven (NL); TIERNEY, Jaime, Eindhoven (NL); LONG, Willie Jie, 5656AG Eindhoven (NL); PRATER, Dave, Eindhoven (NL); RAFTER, Patrick, Eindhoven (NL)
(74) Representative: Philips Intellectual Property & Standards

(57) **Abstract**

A method for medical image compounding in which a compounding method is adaptively varied at different regions of the image frame, as a function of pixel values at or around each pixel location across a received set of image planes to be compounded. In some embodiments, for each of at least a subset of pixels in the compounded image, one of a set of different possible compounding methods is selected, these including maximum intensity pixel selection for regions of highest average pixel value and minimum intensity pixel selection for regions of lowest average pixel value.

## Description

### FIELD OF THE INVENTION

The invention relates to a method of spatial compounding in ultrasound imaging.

### BACKGROUND OF THE INVENTION

Spatial compounding is an established method in ultrasound imaging. This technique aims to reduce speckle noise and improve image contrast. Spatial compounding operates through the incoherent combination of two or more transmit or receive events at differing look directions for the same object.

One type of spatial compounding is elevational spatial compounding. Here, multiple image planes extending in different respective angles in the elevation direction are compounded to form a compound image.

In particular, in ultrasound imaging, the elevation direction refers to the "out of plane" dimension of the ultrasound beam. The dimensions of an ultrasound beam can be described in terms of three directions:
- Azimuthal (lateral) direction: This is the side-to-side width of the beam. This is also known as the transverse or x-axis direction;
- Axial (range or depth) direction: This is the direction of propagation of the beam into the tissue, from the transducer surface downward. It is also known as the y-axis direction.
- Elevation (slice thickness or out-of-plane) direction: This is the front-to-back thickness of the beam. It is perpendicular to the azimuthal and axial directions, and in 2D imaging, it is the direction that is typically not displayed (i.e. in a B-mode image, it is the direction into or out of the image plane). It may also be referred to as the z-axis direction.

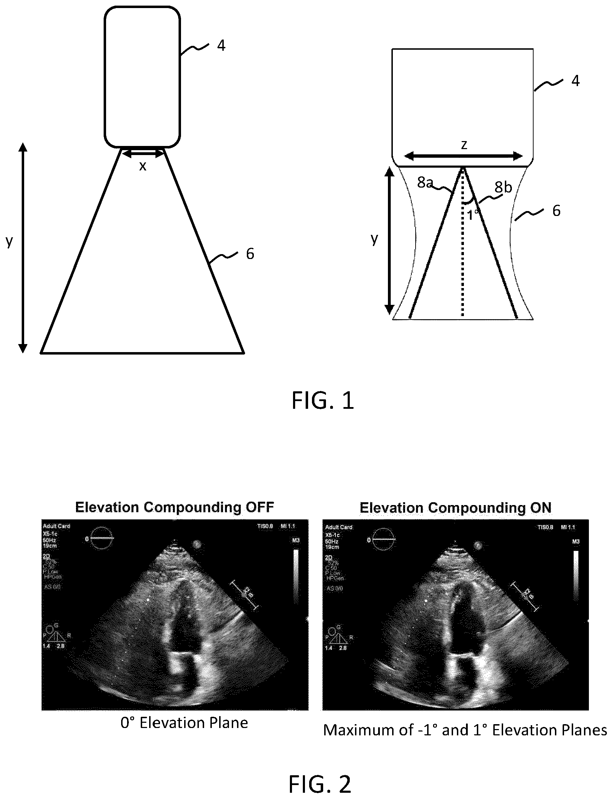

Fig. 1 (left) shows a schematic drawing of a front view of an ultrasound transducer 4, with an ultrasound beam 6 propagating from a transducer array at its lower face. The axial (y) and azimuthal (x) directions are visible in this view. Fig. 1 (right) shows a schematic drawing of a side view of the beam 6. This shows the axial (y) and elevation (z) dimensions of the beam.

In 2D ultrasound, images are often represented in the azimuthal and axial directions, with the elevation direction contributing to slice thickness but not displayed as part of the image. In 3D ultrasound, volumetric images are acquire spanning all three dimensions.

For elevational spatial compounding, multiple imaging planes are captured, each extending in a differing look-direction in the elevation direction. By this is meant that the planes are angled at different orientations in the elevation direction. This is schematically illustrated in Fig. 1 (right) which shows two planes 8a, 8b, the first plane 8a extending at -1° in the elevation direction, and the second plane 8b extending at +1° in the elevation direction.

For imaging employing a 2D array, variable look directions can be achieved by executing elevational parallel receive beamforming at different angles. The resulting B-mode images are then compounded post-log compression. This approach is beneficial due to its requirement of only a singular transmit event in elevation, thereby maintaining frame rates and preventing potential decorrelation of structures in the case of movement of an imaged object (e.g. heart motion).

One example elevational spatial compounding technique employs elevation compounding across two elevation planes at -1° and 1°. Compounding is achieved by taking the maximum value across planes at each pixel. Fig. 2 (right) shows an example compounded image formed from compounding of two elevation planes at -1° and 1°. For comparison, Fig. 2 (left) shows an image of the same structure formed without compounding. This image was acquired at an on-axis 0° elevation plane.

The conventional elevation compounding approach has proven to be an effective solution in improving speckle appearance and, in cardiac imaging, reducing "drop-out".

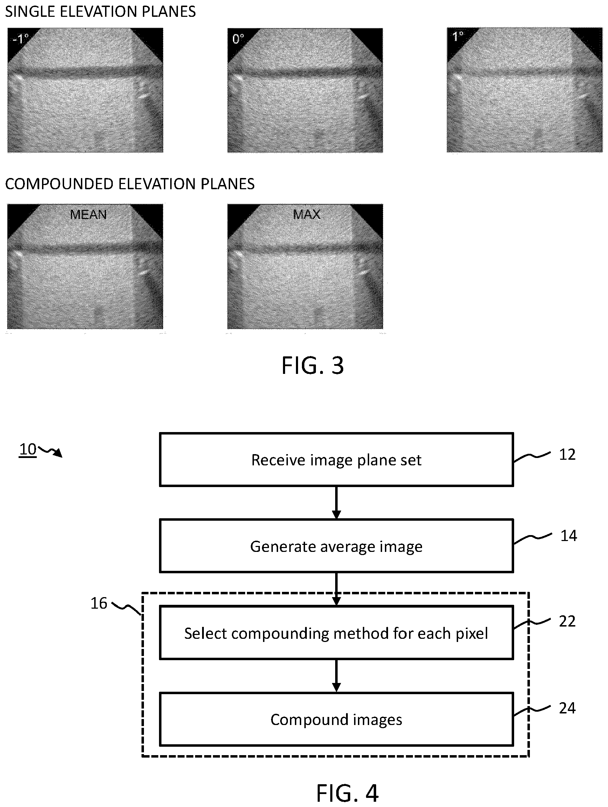

However, while utilizing the maximum value at all pixels aids in smoothing speckle appearance, it also amplifies undesired signal, noise, and clutter within the image. For instance, the contrast of off-axis anechoic structures (such as blood vessels) diminishes when taking the maximum value. In particular, such structures should display as dark (low intensity reflection) within an image. However, the use of maximum intensity compounding results in these structures appearing washed out and reduced in contrast. This is illustrated in Fig. 3. Fig. 3 (top row) shows single image planes of an anechoic vessel in a phantom, each acquired at a different directional angle in the elevation direction. Fig. 3 (top left) shows an image plane oriented at -1° in the elevation direction. Fig. 3 (top middle) shows an image plane oriented at 0° in the elevation direction (i.e. the axial plane). Fig. 3 (top right) shows an image plane oriented at +1° in the elevation direction. For these image planes, no compounding is performed. Fig. 3 (bottom) shows compounded images, each formed by compounding of the three image planes on the top row of Fig. 3 by a different respective compounding approach. Fig. 3 (bottom left) shows a compound image formed from a mean of the three single elevation planes of Fig. 3 (top). Fig. 3 (middle) shows a compound image formed by taking a maximum intensity pixel of the three single-elevation planes at each pixel location.

It can be seen that the vessel exhibits greater visibility in the -1° and 0° elevation planes (top left and top middle) in comparison to the 1° elevation plane (top right). Therefore, despite enhancements to speckle smoothing, the vessel's contrast is worsened by the standard maximum elevation compounding method (bottom middle) as opposed to a method without elevation compounding;

Using the mean value instead of the maximum can improve the contrast of the vessel (as shown in Fig. 3 (bottom left)), but still produces inferior vessel contrast compared to the -1° and 0° images without elevation compounding.

Ideally, for the example provided in Fig. 2 and Fig. 3, the minimum value at pixels within the vessel would be taken to maintain vessel contrast. However, this would then compromise the speckle reduction elsewhere in the image.

An improved spatial compounding method able to overcome the above-described difficulties would be of value.

### SUMMARY OF THE INVENTION

The invention is defined by the claims.

According to examples in accordance with an aspect of the invention, there is provided a method for spatial compounding in ultrasound imaging, comprising:
receiving a set of two or more ultrasound image planes representing planes through an imaged object extending in different respective look directions;
generating a compounded image from the set of two or more ultrasound image planes, wherein each pixel of the compounded image is generated by compounding corresponding pixels in each of the set of two or more received ultrasound image planes.

The generating of the compounded image comprises, for each individual pixel of the compounded image, selecting a compounding method from a pre-defined set of compounding methods, wherein the set includes at least: selecting a maximum of the pixel values for the pixel location from among the set of received image planes; and selecting a minimum of the pixel values for the pixel location from among the set of received image planes.

In some embodiments, for at least a subset of the pixels of the compounded image, the selection may be such that at least pixels of the subset having a lowest average pixel value at a corresponding pixel location or a defined neighborhood thereof across the received set of image planes are compounded using the minimum pixel value compounding method and at least pixels of the subset having a highest average pixel value at a corresponding pixel location or a defined neighborhood thereof across the received set of image planes are compounded using the maximum pixel value compounding method.

In some embodiments, the method comprises generating an average image from the received set of two or more image planes.

In some embodiments, for at least a subset of pixels of the compounded image, the selection may be such that at least pixels of the at least subset falling within lowest intensity pixel regions in the average image are compounded using the minimum pixel value compounding method, and at least pixels of the at least subset in highest intensity pixel regions of the average image are compounded using the maximum pixel value selection method.

In some embodiments, the selecting of the compounding method for each pixel (or each of the at least subset of pixels) may include a step of comparing an average pixel value at the corresponding pixel location, or average pixel values in a defined neighborhood thereof, in the average image against at least one threshold. Additionally or alternatively, a machine learning algorithm may be employed to make the selection.

Embodiments of the invention are thus based on the concept of individually varying a compounding type or method used to compound each pixel of a compounded image. In some embodiments, and for at least a subset of pixels, the compounding method may be chosen based on a value of the average pixel (e.g. mean pixel) of the received image planes at the corresponding pixel location, or average values of pixels in a defined neighborhood of the corresponding pixel location. In other words, the compounding method may be varied across the compound image in dependence upon an average ultrasound reflection intensity at that location of the original images. As explained above, a general objective is to avoid loss of contrast or contrast resolution of anechoic structures in images. It is proposed to do this at least in some embodiments by using the minimum pixel value compounding method in regions of the image where intensity is low (e.g. regions containing anechoic structures) and to using the maximum pixel value compounding method in regions of the image where intensity is high. There are various particular methods for implementing this selection approach, as will become clear from descriptions to follow.

One particularly advantageous application for the method is for elevational spatial compounding in ultrasound imaging. In this case the set of two or more ultrasound image planes represent planes through an imaged object extending in different respective look directions in the elevation direction. However, the method can be used for spatial compounding of image planes which are angularly oriented in any direction. For example, the same method can be used for compounding images in multiplanar reformatting.

The aforementioned at least subset of pixels may be determined in some embodiments at least in part on a variance or spread of pixel values across the image planes at each particular pixel location or a defined neighborhood thereof. For example, the at least subset of pixels may in some embodiments exclude pixels for which a variance of pixel values across the image planes at the pixel location or a defined neighborhood thereof is above a threshold. For such pixels, a different compounding method may be used in accordance with a pre-defined additional rule.

For example, where variance (or another measure of spread) is high, this indicates a large difference between the pixels to be compounded. In those circumstances, the use of maximum or minimum intensity projection may be undesirable given that the pixels to be compounded vary considerably. Selecting a different compounding rule, in accordance with a supplementary rule might be preferred in such circumstances. For example, in the use case of elevational spatial compounding, the 0 degree plane value may be a better choice. This might be added as an additional rule when assigning the compounding methods to each pixel.

The selecting of the compounding method for each pixel may for example be performed by applying a pre-defined selection logic, which may comprise a prioritized sequence of selection rules, where each selection rule is applied provided compliance with all higher priority selection rules.

In some embodiments, the at least subset of pixels includes a majority of pixels of the compounded image.

With regards to the average image, this may be a mean image in some embodiments, wherein each pixel of the mean image is formed by taking a mean of the pixels at the corresponding pixel location in each of the received set of image planes, or a mean of pixel values in a defined neighborhood of the corresponding pixel location in each of the received set of image planes. For example, the defined neighborhood might be the 3x3 square region or 4x4 square region (kernel) around each pixel.

Instead of the mean, any other form of average could be used, e.g. median, mode, weighted mean, truncated mean. In some embodiments, each pixel of the average image might be representative of a variance of pixel values of corresponding pixels in each of the received set of image planes.

In some embodiments, the at least one threshold is an (adaptive) threshold function representing threshold values which vary as a function of depth in the average image.

Intensity of ultrasound echoes naturally diminishes as a function of depth, and thus the threshold used to determine which compounding method to use may be correspondingly decreased as a function of depth.

Additionally or alternatively, in some embodiments, the at least one threshold is an adaptive threshold function representing threshold values which vary as a function of lateral/azimuthal steering angle at which a pixel in the average image was acquired. With regards to the lateral steering angle at which a pixel is acquired, this refers to the angle at which the ultrasound beam is steered or directed perpendicular to its axial direction (left-right when viewing a standard ultrasound image). The signal-to-noise ratio can change depending on the steering angle, largely due to the change in incidence angle of the ultrasound beam with the tissue interfaces. At larger steering angles, the ultrasound waves may not be as efficiently reflected, resulting in weaker signals and reduced image quality. To account for these changes in signal strength and quality, it may be beneficial to adjust the thresholds for the compounding methods based on the lateral steering angle.

In some embodiments, the at least one threshold (e.g. the threshold function) is computed based on pixel values in the average image.

For example, in some embodiments, the computing of the threshold function may comprise: deriving a probability distribution of pixel intensities in the average image, and determining the threshold function based on the probability distribution. For example, in some embodiments, the threshold function may be defined as a function which decays from a starting maximum value, wherein the starting maximum value is based on a pixel value at a pre-defined percentile of the probability distribution, e.g. 90^{th} percentile. This may be a linear function or a non-linear function, for example an exponential decay function.

In some embodiments, the selecting of the compounding method for each pixel of the at least subset of pixels may comprise forming a mask using the at least one threshold and using the average image.

For example, in some embodiments, the selecting the compounding method for each of the at least subset of pixels may comprise forming a binary mask by applying the at least one threshold (e.g. the threshold function) to pixels of the average image, wherein if the average pixel intensity is above the at least one threshold, the mask pixel is assigned a value of 1 in the binary mask and if the intensity is below the threshold, the mask pixel is assigned a value of 0, or vice versa. The selecting of the compounding method for each of the at least subset of pixels may then be performed based on values of the mask.

If the at least one threshold is a threshold function, then each average pixel value is compared against a value of the threshold function corresponding to the location of the pixel. For example, if the threshold function is a function of depth, then each average pixel is compared against a value of the threshold function corresponding to the depth of the relevant average pixel value.

In some embodiments, if the mask has a value of 1, the maximum intensity compounding method is used, and if the value of the mask is 0, the minimum intensity compounding method is used (or vice versa if the mask assignments are the other way around).

In some embodiments, the method may further comprise applying an edge-preserving smoothing filter to the binary mask to convert the binary mask to a non-binary mask before selecting the compounding method for each pixel. In some embodiments, in this case, the defined set of compounding methods may further include: using a mean of the pixel values of the set of received image planes at the pixel location. In this set of embodiments, the following selections for the compounding method may be used: if the value of the non-binary mask at a pixel location is 1, the maximum intensity compounding method is used; if the value of the non-binary mask at a pixel location is 0, the minimum intensity compounding method is used; and if the value of the mask at a pixel location is between 0 and 1, a weighted combination of the set of compounding methods is used.

The chosen weightings may vary proportionally with the mask value.

In some embodiments, the at least one threshold is determined based on application of a trained machine learning algorithm to the received set of two or more image planes.

In some embodiments, the computing of the binary and/or non-binary mask may comprise application of a trained machine learning algorithm to the received set of two or more image planes.

As mentioned briefly above, in some embodiments, the method may be a method for elevational spatial compounding in ultrasound imaging, and wherein the set of two or more ultrasound image planes represent planes through an imaged object extending in different respective look directions in the elevation direction. In elevational spatial compounding, the ultrasound beam is transmitted at different angles in the elevation, or slice-thickness plane, reducing image artefacts and improving the representation of structures.

Spatial compounding can also be done with image planes which vary in look direction across a directional axis which is different to the elevational axis, e.g. the lateral/azimuthal direction.

Another example application for the method is for compounding images in a multiplanar reformatting method. In this case, spatial compounding is used to combine 2D slices of a 3D ultrasound data set to improve the quality of the reformatted image. This is particularly useful in obstetrics and gynecology, where it can provide more detailed images of a fetus or pelvic structures.

In some embodiments, the received set of two or more image planes includes one image plane extending at a look angle of 0° in the elevation direction. In other words, this corresponds to the on-axis 0° elevation plane.

In some embodiments, the received set of two or more image planes includes: one image plane extending at a look angle of -1° in the elevation direction; one image plane extending at a look angle of 0° in the elevation direction; and one image plane extending at a look angle of +1° in the elevation direction.

In some embodiments, the method comprises: receiving ultrasound data of the imaged object generated by an array transducer, the data spanning a volumetric region having an elevation direction, an axial direction and a lateral/azimuthal direction, performing elevational parallel receive beamforming to generate data corresponding to a set of two or more planes extending at different respective look directions in the elevation direction, and processing the generated data for the two or more planes to derive respective B-mode images corresponding to image planes extending in each of the two or more look directions.

The method is a machine-implemented method. The method may be a computer-implemented method. The method is not a mental method.

Another aspect of the invention is a computer program product comprising computer program code configured, when executed by a processor, to cause the processor to perform a method in accordance with any embodiment described in this disclosure, or in accordance with any claim in this application.

The invention can also be embodied in hardware form.

Thus another aspect of the invention is a processing device, comprising one or more processors, configured to: receive a set of two or more ultrasound image planes representing planes through an imaged object extending in different respective look directions; and generate a compounded image from the set of two or more ultrasound image planes, wherein each pixel of the compounded image is generated by compounding corresponding pixels in each of the set of two or more received ultrasound image planes. The generating the compounded image may comprise, for each individual pixel of the compounded image, selecting a compounding method from a pre-defined set of compounding methods, wherein the set includes at least: selecting a maximum of the pixel value for the pixel location from among the set of received image planes; and selecting a minimum of the pixel value for the pixel location from among the set of received image planes.

In some embodiments, for at least a subset of the pixels of the compounded image, the selection may be such that at least pixels of the subset having a lowest average pixel value at a corresponding pixel location or a defined neighborhood thereof across the received set of image planes are compounded using the minimum pixel value compounding method and at least pixels of the subset having a highest average pixel value at a corresponding pixel location or a defined neighborhood thereof across the received set of image planes are compounded using the maximum pixel value compounding method.

In some embodiments, the one or more processors may be further adapted to generate an average image from the received set of two or more image planes.

In some embodiments, for at least a subset of pixels of the compounded image, the selection may be such that at least pixels of the at least subset falling within lowest intensity pixel regions in the average image are compounded using the minimum pixel value compounding method, and at least pixels of the at least subset in highest intensity pixel regions of the average image are compounded using the maximum pixel value selection method.

In some embodiments, the selecting of the compounding method for each pixel (or each of the at least subset of pixels) may include a step of comparing an average pixel value at the corresponding pixel location, or average pixel values in a defined neighborhood thereof, in the average image against at least one threshold. Additionally or alternatively, a machine learning algorithm may be employed to make the selection.

Another aspect of the invention is a system comprising: an ultrasound imaging apparatus; and the processing device in accordance with any embodiment in this disclosure or any claim of this application. The processing device is operatively coupled with the ultrasound imaging apparatus, and adapted for receiving ultrasound data generated by the ultrasound imaging apparatus.

The ultrasound imaging apparatus may comprise an ultrasound probe which includes an ultrasound transducer array, for example a 1.5D, 1.75D, or 2D transducer array.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates the elevation, azimuthal and axial dimensions of an ultrasound beam;
Fig. 2 illustrates the effect of a known elevational spatial compounding method on image quality;
Fig. 3 illustrates different image quality in single elevation planes compared with two compound images formed with two known compounding methods;
Fig. 4 outlines steps of an example method in accordance with one or more embodiments of the invention;
Fig. 5 is a block diagram of an example processing device and system in accordance with one or more embodiments of the invention;
Fig. 6 shows a flow diagram illustrating the process for forming the compounded image in accordance with one preferred set of embodiments;
Fig. 7 illustrates an example thresholding function according to one or more embodiments;
Fig. 8 illustrates the effect of elevational compounding on image contrast using different methods for compounding the pixels of the component images;
Fig. 9 illustrates a difference between a compounded image formed entirely from a single compounding method (maximum intensity projection) and a compounded image formed using the adaptive compounding approach of an embodiment of the present invention; and
Fig. 10 shows comparative images generated using a single compounding method (maximum intensity projection), and using two different adaptive compounding methods in accordance with embodiments of the present invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for medical image compounding in which a compounding method is adaptively varied at different regions of the image frame, as a function of reflection intensity at or around each pixel location across the set of image planes to be compounded. For each pixel in the compounded image, one of a set of different possible compounding methods is selected. In at least some embodiments, and for at least a subset of pixels, these may include maximum intensity pixel selection for regions of highest average pixel value and minimum intensity pixel selection for regions of lowest average pixel value. This yields a compounded image with improved contrast, particularly for anechoic structures, as compared with a compounded image formed using a single compounding method for all pixels.

Fig. 4 outlines in block diagram form steps of an example method according to one or more embodiments. The steps will be recited in summary, before being explained further in the form of example embodiments.

The method 10 is for spatial compounding in ultrasound medical imaging. As discussed above, compounding in the context of ultrasound imaging refers to the process of combining multiple ultrasound image planes oriented at different angles (or look directions) to form a single image.

The method 10 comprises receiving 12 a set of two or more ultrasound image planes representing planes through an imaged object extending in different respective look directions. The term "look direction" refers to the orientation or direction in which the image plane is angled. It might otherwise be referred to as the plane orientation of the ultrasound plane.

The method may further comprise generating 14 an average image from the received set of two or more image planes. An average image is derived for example by computing the average pixel values from each corresponding pixel location across all the received ultrasound image planes, or computing average pixel values in a defined neighborhood around each corresponding pixel location across all the received ultrasound image planes. The average may be the mean in some examples. For example, if there are three image planes, the value of a specific pixel in the average image may be calculated by adding the values of the corresponding pixel in each of the three planes and dividing the total by three. Alternatively, for each pixel location, it might be computed by taking an average of pixel values in each image plane within a neighborhood of the pixel location, e.g. a 3x3 or 4x4 square neighborhood, and then taking an average of these averages across the three image planes.

The process may be repeated for all pixels, resulting in an average image that represents the average intensity of the image planes. Instead of using the mean, a different averaging method might be used. A weighted average might be used in some examples. The variance (or another measure of similarity or spread) of the pixels of the received set of image planes might be used in some examples, thereby yielding a variance image instead of a mean image.

However, the average is determined for each pixel location, the pixels of the final average image may represent those averages, or correlate values thereof. For example, the average image may be an image which is a function of, or is correlated with, an average of the received set of image planes.

The method further comprises generating 16 a compounded image from the set of two or more ultrasound image planes. Each pixel of the compounded image is generated by compounding corresponding pixels in each of the set of two or more received ultrasound image planes.

The generating 16 the compounded image comprises, for each individual pixel of the compounded image, selecting 22 a compounding method from a pre-defined set of compounding methods, wherein the set includes at least: selecting a maximum of the pixel values for the pixel location from among the set of received image planes; and selecting a minimum of the pixel values for the pixel location from among the set of received image planes.

In some embodiments, for at least a subset of the pixels, the selection 22 may be such that at least pixels of the subset having a lowest average pixel value at a corresponding pixel location or a defined neighborhood thereof across the received set of image planes are compounded using the minimum pixel value compounding method and at least pixels of the subset having a highest average pixel value at a corresponding pixel location or a defined neighborhood thereof across the received set of image planes are compounded using the maximum pixel value compounding method.

In some embodiments, for at least a subset of pixels, the selection 22 may be such that at least pixels of the subset falling within lowest intensity pixel regions in the average image are compounded using the minimum pixel value compounding method, and at least pixels of the subset in highest intensity pixel regions of the average image are compounded using the maximum pixel value selection method. Accordingly, regions of the imaged object which are low intensity (e.g. anechoic objects such as blood vessels) are rendered in the compounded image with the minimum intensity pixel value and objects which are higher intensity are rendered with the maximum intensity pixel value. Accordingly, image contrast is improved compared with a compounding method that uses the same compounding method for all pixels.

In some embodiments, the selecting 22 of the compounding method for each pixel (or each of the at least subset of pixels) may include a step of comparing an average pixel value at the corresponding pixel location, or average pixel values in a defined neighborhood thereof, in the average image against at least one threshold. Additionally or alternatively, the selecting step may be performed by a machine learning algorithm, for example applied to the average image as in input, and/or applied to the set of received image planes as input.

The aforementioned at least subset of pixels may be determined in some embodiments in a separate step. For example, the method may comprise a step of determining the at least subset of pixels based on a statistical or other measure of pixel values at each pixel location across the set of received image planes. For example, the method may comprise determining the at least subset of pixels based in part on a variance or spread of pixel values across the image planes at each particular pixel location, or a defined neighborhood thereof. For example, the at least subset of pixels may in some embodiments exclude pixels for which a variance of pixel values across the image planes at the pixel location or a defined neighborhood thereof is above a threshold. For such pixels, a different compounding method may be used in accordance with a pre-defined additional rule.

For example, where variance (or another measure of spread) is high, this indicates a large difference between the pixels to be compounded. In those circumstances, the use of maximum or minimum intensity projection may be undesirable given that the pixels to be compounded vary considerably. Selecting a different compounding rule, in accordance with a supplementary rule, might be preferred in such circumstances. For example, in the use-case of elevational spatial compounding, the 0 degree plane value may be a better choice. This might be added as an additional rule when assigning the compounding methods to each pixel.

In some embodiments, the at least subset of pixels includes a majority of pixels of the compounded image.

More generally, it may be understood that the selection of the compounding method for each pixel may in some embodiments comprise application of a pre-defined selection logic, which may comprise a sequence of selection rules, each applying to a subset of the pixels of the compounded image in accordance with criteria. This may for example comprise a hierarchical or prioritized sequence of selection rules, where each selection rule is applied to a given pixel provided compliance with all higher priority selection rules.

The average pixel value, as mentioned above, may for example correspond to the average value of a specific pixel across all image planes, or for example an average value of pixels in a neighborhood around a specific pixel, averaged across all image planes. As discussed above, the threshold against which this average pixel value is compared can be determined in a number of different ways, and the threshold comparison may in some preferred embodiments take place as part of a more complex selection process. For example, the comparison against a threshold may be used to form a mask, and wherein the mask is then used to select the compounding method. The threshold(s) may be set to distinguish between different types of structures or regions in the image, typically based on their intensity. For instance, higher intensity regions might represent certain tissues or structures, while lower intensity regions might represent different tissues or fluid-filled areas.

The received images 12 are compounded 24 based on the determined compounding method for each pixel.

As noted above, the method can also be embodied in hardware form, for example in the form of a processing device which is configured to carry out a method in accordance with any example or embodiment described in this document, or in accordance with any claim of this application.

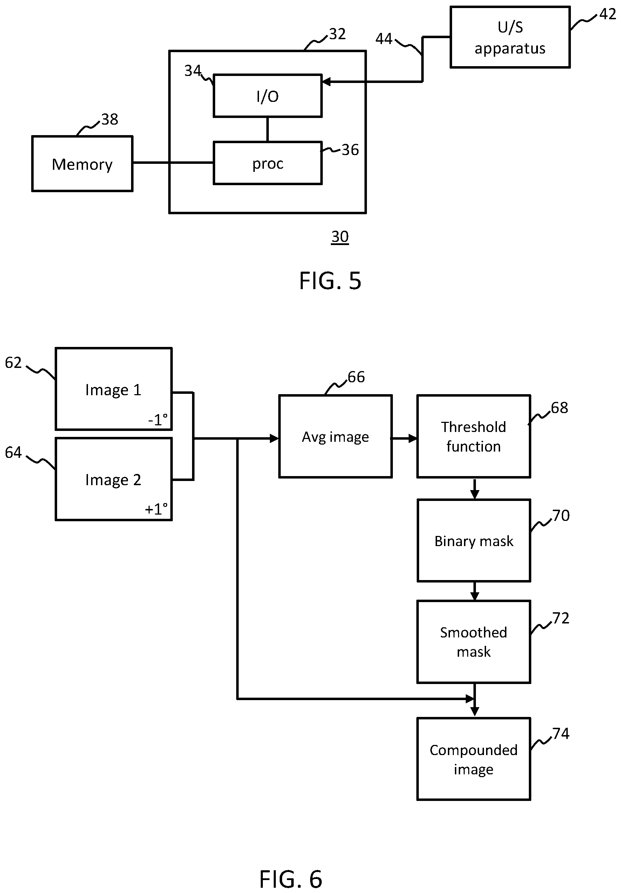

To further aid understanding, Fig. 5 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 5, the system 30 further comprises an ultrasound acquisition or imaging apparatus 42 for acquiring 2D or 3D ultrasound imaging data 44. However, this is optional. The ultrasound imaging apparatus may comprise an ultrasound probe which includes an ultrasound transducer array, for example a 1.5D, 1.75D, or 2D transducer array.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

An example implementation of the method according to a particular set of embodiments will now be described by way of illustration of the above-summarized concept of the invention. It will be appreciated that not all features of this particular set of embodiments are essential to the inventive concept, and are described to aid understanding and to provide an example to illustrate the inventive concept. In this set of embodiments, the compounding method is a method for elevational spatial compounding in ultrasound imaging, and wherein the set of two or more ultrasound image planes represent planes through an imaged object extending in different respective look directions in the elevation direction. However, the same described method can be applied for compounding a plurality of image planes with any variation in planar orientation.

Fig. 6 shows a flow diagram which illustrates the main steps of the example method according to the presently described set of embodiments.

The method begins with receipt of the set of two or more ultrasound image planes representing planes through an imaged object extending in different respective look directions. In this example, for illustration, the set comprises two image planes: a first image plane 62 and a second image plane 64. The first image plane 62 is an image plane extending at a look angle of -1° in the elevation direction. The second image plane 64 is a plane extending at a look angle of +1°in the elevation direction. In some embodiments, the set may additionally include a third image plane extending at a look angle of 0°in the elevation direction.

An average image 66 is computed from the received set of image planes 62, 64, as mentioned previously. Preferably, the average image is a mean image, wherein each pixel of the mean image represents a mean of the pixel values at the corresponding pixel location in each of the received image planes 62, or a defined neighborhood thereof.

A set of intensity-based thresholds are then computed from the average image. In other words, the at least one threshold referenced previously comprises a plurality of thresholds and these are computed based on pixel values in the average image. More particularly, in this set of embodiments, a threshold function is computed, which defines threshold value as a function of pixel location. In some embodiments, the values of the threshold function may vary as a function of depth of the pixel in the average image. Additionally or alternatively, the threshold function may vary as a function of the lateral/azimuthal steering angle at which a pixel in the image was acquired.

To explain, as ultrasound waves propagate into the tissue, the waves are attenuated as a function of depth, such that weaker signals are reflected back to the transducer from deeper structures. This results in a decrease in image brightness with increasing depth. When generating the image planes, it is common for gain to be increased as a function of depth to compensate for the attenuation effect (using Time-Gain Compensation, TGC). This has a consequence that the signal-to-noise ratio tends to decrease as a function of depth because both the actual signal and the noise are amplified. Hence, to account for these changes, the thresholds used for the compounding methods may be adapted as a function of depth. For example, to ensure adequate differentiation of structures at different depths, a higher threshold may be applied at deeper regions as compared to superficial regions.

With regards to the lateral steering angle at which a pixel is acquired, this refers to the angle at which the ultrasound beam is steered or directed perpendicular to its axial direction (left-right when viewing a standard ultrasound image). The signal-to-noise ratio can change depending on the steering angle, largely due to the change in incidence angle of the ultrasound beam with the tissue interfaces. At larger steering angles, the ultrasound waves may not be as efficiently reflected, resulting in weaker signals and reduced image quality. To account for these changes in signal strength and quality, it may be beneficial to adjust the thresholds for the compounding methods based on the lateral steering angle.

For simplicity, in the presently described examples, a threshold function 68 is assumed to be computed which varies solely as a function of depth in the images. Since the threshold varies as a function of pixel location, it may be referred to as an adaptive threshold or adaptive threshold function. Its application is known as 'adaptive thresholding'.

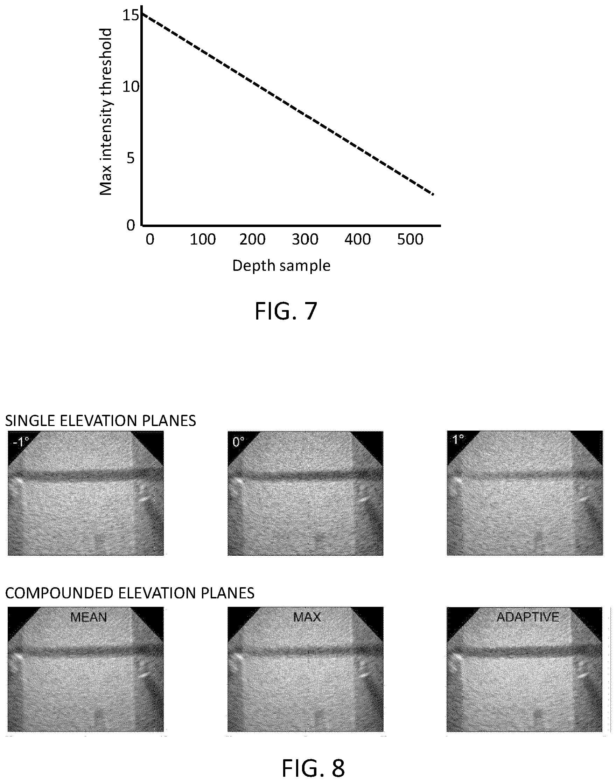

To generate the threshold function from the average image, one possible process is to derive a probability distribution of pixel intensities in the average image, and then define the thresholding function as a function which decays from a starting maximum value, wherein the starting maximum value is based on a pixel value at a pre-defined percentile of the probability distribution, e.g. 90^{th} percentile. In this case, there are two main parameters of the threshold function that can be tuned: the starting maximum percentile value and the slope of the threshold curve. One example thresholding function is illustrated in Fig. 7. In this example, the threshold function is a linear function, but a non-linear function could also be considered, e.g. an exponential decay function. In the illustrated example, the 90th percentile of the distribution of pixel values in the average image 66 is used as the maximum intensity threshold at the shallowest depth. An attenuation factor of 0.55dB/cm/MHz was used to compute the slope and intensity thresholds at deeper depths. These values can of course be adjusted per clinical application to either favor taking the maximum or minimum as the compounding method.

The threshold function may have a pre-defined shape or mathematical form, and wherein only the starting maximum value is configured during execution of the method itself. The shape or curve might be determined based on an estimation of signal loss as a function of depth. For example, this could be tuned to the particular ultrasound imaging equipment, based on estimating signal-to-noise as a function of depth in a batch of acquired images and then setting the threshold function to decline with a shape and rate which matches the drop off in average signal-to-noise in the images.

Returning to the method of Fig. 6, once the threshold function is determined, it is used to compute a binary mask 70 for the average image, wherein intensities above the threshold are set to 1 and those below are set to 0.

More particularly, the method comprises forming a binary mask by applying the threshold function 68 to pixels of the average image 66, wherein if the average pixel intensity at a pixel location is above the value of the threshold function corresponding to that pixel location, then the mask pixel is assigned a value of 1 in the binary mask and if the intensity is below the threshold, the mask pixel is assigned a value of 0 (or vice versa).

In some embodiments, the binary mask 70 could be used alone to determine the compounding methods for each pixel. For example, if the mask has a value of 1, the maximum intensity compounding method is used, and if the value of the mask is 0, the minimum intensity compounding method is used.

Alternatively, and as illustrated in Fig. 6, the method may further comprise applying an edge-preserving smoothing filter 72 (e.g. Kuwahara filter) to the binary mask 70 to convert the binary mask to a non-binary mask before selecting the compounding method for each pixel. The non-binary mask has values which can vary continuously between 0 and 1.

The smoothed mask may then be used to adaptively compound the elevation planes such that, at each pixel, the maximum is taken when the mask is equal to 1, the mean is taken when the mask is between 0 and 1, and the minimum is taken when the mask is equal to 0.

Instead of taking the mean when the mask is between 0 and 1, instead a weighted combination of the set of compounding methods might be used, with the weightings varying in proportion with the mask pixel value.

The final compounded image 74 may be output, for example to a display and/or datastore.

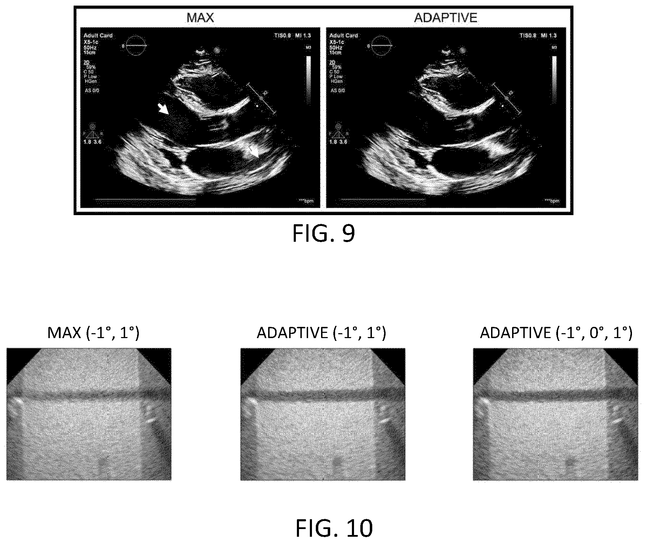

The benefits of the proposed adaptive compounding approach in comparison to the default maximum-pixel compounding approach are demonstrated in Fig. 8 and Fig. 9.

Fig. 8 shows a set of image planes, each corresponding to a different respective look-direction in the elevation direction. Fig. 8 (top left) shows an image plane oriented at -1° in the elevation direction. Fig. 8 (middle) shows an image plane oriented at 0° in the elevation direction (i.e. the axial plane). Fig. 8 (top right) shows an image plane oriented at +1° in the elevation direction. For these image planes, no compounding is performed.

Fig. 8 (bottom) shows compounded images, each formed by compounding of the three image planes on the top row of Fig. 8 by a different respective compounding approach. In Fig. 8 (bottom left), the mean intensity pixel compounding method was used for every pixel in the image. In Fig. 8 (middle), the maximum intensity pixel compounding method was used for every pixel in the image. In Fig. 8 (right), the adaptive compounding method in accordance with an embodiment of the present invention was used.

Fig. 8 shows how the adaptive approach (bottom right) better preserves the anechoic vessel seen in the single elevation plane images (Fig. 8 (top)) while also maintaining the speckle-smoothing benefits of compounding seen in the default global maximum image (Fig. 8 (middle)).

Fig. 9 demonstrates the feasibility of the proposed adaptive compounding approach. Fig. 9 (left) shows a compounded image formed using the standard maximum intensity pixel compounding approach for every pixel. Fig. 9 (right) shows a compounded image formed using the adaptive compounding method in accordance with one or more embodiments of the present invention. The adaptive compounded image (Fig. 9 (right)) shows that the adaptive approach yields similar speckle appearance to the default approach while also suppressing unwanted signal enhancement in anechoic regions in the image. In particular, white arrows in Fig. 9 (left) point to regions of unwanted signal in anechoic regions in the image. These are better suppressed in the adaptively compounded image of Fig. 9 (right).

As discussed above, one application for the adaptive compounding method according to embodiments of the invention is for elevational spatial compounding. In this context, one relevant consideration is the number of elevation planes to be used and which elevational angles to use.

According to one set of embodiments, the received set of ultrasound image planes may include one image plane extending at a look angle of -1° in the elevation direction and one image plane extending at a look angle of +1°in the elevation direction.

Further to this, it has been found that including the on-axis 0° elevation plane often improves the adaptive approach. This is exemplified in Fig. 10. This shows a set of compounded images. Fig. 10 (left) shows a compounded image formed from compounding of a -1° and +1° elevational plane using a standard maximum intensity compounding approach. Fig. 10 (middle) shows a compounded image formed from compounding of a -1° and +1° elevational plane using the adaptive compounding approach according to an embodiment of the present invention. Fig. 10 (right) shows a compounded image formed from compounding of a -1°, 0°, and +1° elevational plane using the adaptive compounding approach according to an embodiment of the present invention.

Including the on-axis 0° elevation plane for the adaptive approach (Fig. 10 (right)) in this example results in improved vessel contrast compared to the adaptive approach using only the -1° and 1° elevation planes (Fig. 10 (middle)).

As a variation on the proposed method, in some advantageous embodiments, the creation, smoothing, and application of the mask may potentially be improved and optimized for different applications. In the example embodiment described above, the binary mask 70 was generated based on thresholds applied to intensities of pixels in the average image 66. As an alternative, in some embodiments, data-driven machine learning techniques could potentially be used to generate a mask.

Thus, to state this more precisely, in some embodiments, the at least one threshold (e.g. the threshold function) may be determined based on application of a trained machine learning algorithm to the received set of two or more image planes. Additionally or alternatively, the computing of the binary and/or non-binary mask may comprise application of a trained machine learning algorithm to the received set of two or more image planes. More generally, the selecting 22 of the compounding method to be used for each pixel in the compounded image may be performed using a machine learning algorithm. In practice, this might be achieved by using a machine learning algorithm to generate a mask which classifies each pixel location in terms of a compounding method to be used for forming that pixel in the compounded image.

By way of example, in some embodiments, an artificial neural network may be trained to receive an input set of image planes (or an input average image) and to generate as output a mask comprising a classification for each mask pixel indicative of a selected one of the set of possible compounding methods. This type of task is known generally in the field as sematic segmentation, which involves classifying each pixel in an image to a particular class. One type of machine learning model that would be suitable for this task is a Convolutional Neural Network (CNN), for example a CNN utilizing U-Net architecture.

The training data used in training the machine learning model may comprise a plurality of training image sets, each training image set comprising a set of images of a same object acquired at different orientation angles (look angles), and wherein each training image set is further accompanied by a ground truth mask indicating a classification of each pixel. In particular, the masks may indicate the compounding method for each pixel. The U-Net model is typically trained using a pixel-wise loss function, such as cross-entropy or Dice loss. During training, the model will learn to associate specific image features with specific compounding methods. It will use this learned knowledge to predict the compounding method for each pixel in new, unseen images.

Each ground truth mask might be manually prepared, for example based on a human identifying pixels which lie within anechoic structures and determining classifications for each pixel based thereon. This represents just one example approach. Instead of the ground truth mask being prepared by a human, it might be prepared using an automated segmentation algorithm, wherein structures in the image are segmented, and then the algorithm assigns compounding methods to each pixel based on where it lies in relation to the segmented structures.

It might be valuable to use a machine learning approach wherein analysis of the input images is based not only on values of individual pixels but also pixels in the surrounding neighborhood. This may potentially produce a more coherent mask. To achieve this, one possibility might be to use a Fully Convolutional Network (FCN). FCNs preserve spatial information throughout the entire network, which allows for the consideration of the context of each pixel, not just the pixel itself. Alternatively, in U-Net, a similar result can be achieved by appropriately setting the receptive field of the convolutional filters and the depth of the network. The receptive field refers to the region in the input space that a particular CNN's feature is looking at. A larger receptive field implies that the network is capable of incorporating more context into predictions.

In addition to or instead of using thresholding of intensity of pixels in the received images to determine a compounding method for each pixel location, a degree of similarity of pixels at a same pixel location across a set of received image planes could be taken into account. For example a variance of pixels at a same pixel location, or a correlation measure between pixels at a same pixel location. Spatial correlation is a statistical measure of how similar pixels at corresponding pixel locations in two or more images are to each other. With regards to variance, in areas where there is a large variance it indicates a large difference between the pixels to be compounded. In those circumstances, the use of maximum or minimum intensity projection may be undesirable given that the pixels to be compounded vary considerably. Selecting the 0 degree plane value may be a better choice under those circumstances. This might be added as an additional rule when assigning the compounding methods to each pixel. For example, the intensity-based mask might be used by default, unless a similarity measure between the pixels across the set of received image planes at a certain pixel location is below a threshold, in which case a pixel value from the 0 degree plane is used (or another pre-determined one of the received planes, or a certain computed image based on the received planes).

Certain embodiments discussed above apply smoothing to a binary mask to create a non-binary mask. In this regard, according to a variant set of embodiments, in addition or instead of applying smoothing to the binary mask, smoothing could be applied to the individual received image planes 62, 64, prior to mask creation.

In embodiments in which elevational compounding is being performed, the method may comprise steps for extracting the set of image planes from a received 3D ultrasound dataset. For example, the method may comprise: receiving ultrasound data of the imaged object generated by an array transducer, the data spanning a volumetric region having an elevation direction, an axial direction and a lateral/azimuthal direction. The method may comprise performing elevational parallel receive beamforming to generate data corresponding to a set of two or more planes extending at different respective look-directions in the elevation direction. The method may further comprise processing the generated data for the two or more planes to derive respective B-mode images corresponding to image planes extending in each of the two or more look-directions.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and
EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for spatial compounding in ultrasound imaging, comprising:
receiving (12) a set of two or more ultrasound image planes (62, 64) representing planes through an imaged object extending in different respective look directions;
generating (14) an average image (66) from the received set of two or more image planes;
generating (16) a compounded image (74) from the set of two or more ultrasound image planes, wherein each pixel of the compounded image is generated by compounding corresponding pixels in each of the set of two or more received ultrasound image planes;
wherein the generating the compounded image comprises, for each individual pixel of the compounded image, selecting (22) a compounding method from a pre-defined set of compounding methods, wherein the set includes at least: selecting a maximum of the pixel values for the pixel location from among the set of received image planes; and selecting a minimum of the pixel values for the pixel location from among the set of received image planes;
wherein, for each of at least a subset of the pixels of the compounded image, the selecting of the compounding method for each pixel includes a step of comparing an average pixel value at the corresponding pixel location, or average pixels values in a defined neighborhood thereof, in the average image against at least one threshold (68), and wherein, for the at least subset of the pixels, the selection is such that, at least pixels of the at least subset falling within lowest intensity pixel regions in the average image are compounded using the minimum pixel value compounding method, and at least pixels of the at least subset in highest intensity pixel regions of the average image are compounded using the maximum pixel value selection method.

2. The method of claim 1, wherein the at least one threshold is an adaptive threshold function (68) representing threshold values which vary as a function of depth in the average image and/or as a function of lateral/azimuthal steering angle at which a pixel in the average image was acquired.

3. The method of claim 1 or 2, wherein the at least one threshold is computed based on pixel values in the average image.

4. The method of claim 2 and claim 3, wherein the computing the threshold function comprises:
deriving a probability distribution of pixel intensities in the average image;
defining the thresholding function as a function which decays from a starting maximum value, wherein the starting maximum value is based on a pixel value at a pre-defined percentile of the probability distribution, e.g. 90^{th} percentile.

5. The method of any preceding claim, wherein the selecting of the compounding method for each of the at least subset of pixels comprises:
forming a binary mask (70) by applying the at least one threshold to pixels of the average image, wherein if the average pixel intensity is above the at least one threshold, the mask pixel is assigned a value of 1 in the binary mask and if the intensity is below the threshold, the mask pixel is assigned a value of 0, or vice versa; and
wherein selecting of the compounding method for each of the at least subset of pixels is performed based on values of the mask.

6. The method of claim 5, wherein if the mask has a value of 1, the maximum intensity compounding method is used, and if the value of the mask is 0, the minimum intensity compounding method is used.

7. The method of claim 5,
wherein the method further comprises applying an edge-preserving smoothing filter to the binary mask to convert the binary mask to a non-binary mask (72) before selecting the compounding method for each pixel;
wherein the defined set of compounding methods further includes: using a mean of the pixel values of the set of received image planes at the pixel location, and wherein,
if the value of the non-binary mask at a pixel location is 1, the maximum intensity compounding method is used,
if the value of the non-binary mask at a pixel location is 0, the minimum intensity compounding method is used,
if the value of the mask at a pixel location is between 0 and 1, a weighted combination of the set of compounding methods is used.

8. The method of any preceding claim, wherein the at least one threshold is determined based on application of a trained machine learning algorithm to the received set of two or more image planes.

9. The method of any of claims 5-7, wherein the computing of the binary and/or non-binary mask comprises application of a trained machine learning algorithm to the received set of two or more image planes.

10. The method of any preceding claim, wherein the method is a method for elevational spatial compounding in ultrasound imaging, and wherein the set of two or more ultrasound image planes represent planes through an imaged object extending in different respective look directions in the elevation direction, and preferably wherein the received set of two or more image planes includes one image plane extending at a look angle of 0° in the elevation direction.

11. The method of any preceding claim, wherein said at least subset of pixels is determined based at least in part on a variance or spread of pixel values across the image planes at each particular pixel location or a defined neighborhood thereof, and preferably wherein the at least subset of pixels excludes pixels for which a variance or spread of pixel values across the image planes at the pixel location or a defined neighborhood thereof is above a threshold.

12. The method of any preceding claim, wherein the method comprises:
receiving ultrasound data of the imaged object generated by an array transducer, the data spanning a volumetric region having an elevation direction, an axial direction and a lateral/azimuthal direction,
performing elevational parallel receive beamforming to generate data corresponding to a set of two or more planes extending at different respective look directions in the elevation direction,
processing the generated data for the two or more planes to derive respective B-mode images corresponding to image planes extending in each of the two or more look-directions.

13. A computer program product comprising computer program code configured, when executed by a processor, to cause the processor to perform a method in accordance with any of claims 1-12.

14. A processing device (32), comprising one or more processors (36), configured to:
receive a set of two or more ultrasound image planes representing planes through an imaged object extending in different respective look directions in the elevation direction;
generate an average image from the received set of two or more image planes;
generate a compounded image from the set of two or more ultrasound image planes,
wherein each pixel of the compounded image is generated by compounding corresponding pixels in each of the set of two or more received ultrasound image planes.
wherein the generating the compounded image comprises, for each individual pixel of the compounded image, selecting a compounding method from a pre-defined set of compounding methods, wherein the set includes at least: selecting a maximum of the pixel value for the pixel location from among the set of received image planes; and selecting a minimum of the pixel value for the pixel location from among the set of received image planes;
wherein, for each of at least a subset of the pixels of the compounded image, the selecting of the compounding method for each pixel includes a step of comparing an average pixel value at the corresponding pixel location, or average pixel values in a defined neighborhood thereof, in the average image against at least one threshold, and wherein, for the at least subset of pixels, the selection is such that at least pixels of the at least subset falling within lowest intensity pixel regions in the average image are compounded using the minimum pixel value compounding method, and at least pixels of the at least subset in the highest intensity regions of the average image are compounded using the maximum pixel value selection method.

15. A system (30) comprising:
an ultrasound imaging apparatus (42); and
the processing device (32) of claim 14, operatively coupled with the ultrasound imaging apparatus, and adapted for receiving ultrasound data (44) generated by the ultrasound imaging apparatus.
